# EUROPEAN PATENT APPLICATION

(11) **EP 2 417 986 A1**
(43) Date of publication of application: **15.02.2012**
(21) Application number: 11005652.0
(22) Date of filing: 18.11.2005
(51) Int. Cl.: A61K 47/02, A61K 31/7048, A61K 38/40, A61K 47/12, A61K 47/42, A61P 1/04

(54) **Bioactive compositions**

(30) Priority: 22.11.2004 US 629559 P
(62) Divisional of application: 05817856.7
(71) Applicant: Anadis Ltd., Campbellfield, Victoria 3061 (AU)
(72) Inventor: Rawlin, Grant, Thomas, Kilmore East, Victoria 3764 (AU); Lichti, Gottfried, Essendon, Victoria 3040 (AU); Robins-Browne, Roy, Michael, Templestowe, Voctoria 3106 (AU)
(74) Representative: Schüssler, Andrea

(57) **Abstract**

This invention relates to a bioactive composition comprising: (d) pH sensitive bioactive agent (e) an edible carboxylic acid containing moiety and (f) an edible alkalising moiety, wherein the proportion of said moieties and active agent provide pH control such that (i) when 400mg of said composition is added to 20 ml of 0.033 normal hydrochloric acid and at a temperature of 37 +/- 3 DEG C, the pH reaches a value in the range 4 to 8, and (ii) when 400mg of said composition is added to 20 ml of deionised water at pH 7 and at a temperature of 37 +/- 3 DEG C, the pH reaches a value less than 8.5.

## Description

### Field

The invention relates to bioactive compositions, and in particular relates to bioactive compositions which are pH sensitive and need to act in or traverse through a gastric environment, in order to provide benefits for a wide population of individuals.

Examples of oral bioactive compositions include bovine colostrum (which can be used to relieve the symptoms of gastrointestinal diseases), colostral IgG fraction, hyperimmune colostrum, hyperimmune egg yolk material and other materials described in International Patent Application PCT/AU03/00348 (published as WO 03/080082), which is incorporated by reference.

There are several challenges associated with the use of pH sensitive bioactive compositions. The gastric environment varies greatly between individuals and at different times during the day in the same individual. For example a fasted gastric environment which may occur late in the night or before breakfast may have a pH in the range 1.5 to 2, whereas a post-prandial gastric environment may have a pH in the range 2 to 5 or even higher. Furthermore, in individuals with arrested gastric secretion resulting from age or medication, the gastric pH may be in the range 6 to 7. In addition, the resting volume of gastric fluid in an individual can vary between 5 and 40 ml or even more widely.

A number of strategies have been proposed to improve the efficacy of pH sensitive bioactives.

The following discussion of documents, acts, materials, devices, articles and the like is included in this specification solely for the purpose of providing a context for the present invention. It is not suggested or represented that any or all of these matters formed part of the prior art base or were common general knowledge in the field relevant to the present invention before the priority date of each claim of this application.

US Patent No. 6569453 (Linder and Dietrich) filed in 2001, describes an administration form for acid - labile active compounds including a proton pump inhibitor such as Omeprazole. The formulations have no enteric layers and are suitable for oral administration - they may comprise a sterol such as cholesterol in combination with a polymer such as polyvinyl acetate or PVP/vinyl acetate co-polymer. The formulations may be made by dissolving sterol and polymer in a suitable solvent, suspending the acid labile proton pump inhibitor there-in and spray drying the resulting suspension.

Another strategy is to provide an acid labile active compound with an enteric coating which is rapidly dissolved in the alkaline medium of the intestine after gastric passage. Such a strategy is adopted in European Patent Publication EP-A-0 005 129, EP-A-0 166 287, EP-A-0 174 726 and EP-A-0 268 956. In adopting this strategy the active ingredient must frequently be provided in the form of its alkaline salt, or together with alkaline substances. The substances of use in making enteric coatings are typically those having free carboxyl groups, and in the presence of an alkaline moiety in the interior of the dosage form, dissolution of the coating can take place from the inside out. Free carboxyl groups may promote the decomposition of the active compound. It may therefore be necessary to provide an insulating intermediate layer between the coating and the core material. Linder and Dietrich address this problem by performing a matrix encapsulation to provide protection against harsh acid conditions. The active compound needs to be provided in dry form and comes into contact with non-aqueous solvent. The above features increase cost and solvent use and can lead to denaturing of many bioactive materials, particularly denaturing of the tertiary structure of peptide based materials.

US Patent No. 6312712 (Whittle et al) filed in 2000, teaches that the bioavailability of certain pharmaceutically active moieties can be increased by administering said moieties in combination with a cyclodextrin. The use of this method is limited to active moieties which undergo appropriate reactions with the cyclodextrin for example to form inclusion complexes.

US Patent No. 5998216 (O'Donnell) filed in 1996, describes stabilising formulations for preserving the integrity of proteins present in a body fluid. An important ingredient in the stabilising formulation is a water soluble, high-potency buffering compound. The buffer capacity can be established by measuring the pH change caused by the addition of increments of strong acid or strong alkali to the buffer. High potency soluble buffering compounds include TRIS, di-basic phosphate/mono-basic phosphate, sodium bicarbonate and triethanolamine. O'Donnell refer to a range of body fluids including blood, saliva, pleural fluid, gastric fluid, ascites fluid and synovial fluid.

Many of the buffers referred to by O'Donnell cannot be used over a long course of treatment because of adverse side-effects.

US Patent No 5851579 (Wu et al) filed in 1997, describe an aqueous enteric coating composition comprising an alkali soluble acrylic latex polymer and an aqueous solution of ammonium or alkaline salts of cellulose polymers. Enteric coating strategies such as these have been found to be problematic with many peptide based bioactive materials.

US Patent No. 6468959 (Wunderlich et al) filed in 1994, describes a dosage form for peptides such as insulin. The dosage form comprises a matrix of gelatin or gelatin derivatives having distributed there-in the peptide pharmaceutical. The gelatin derivatives carry a sufficient contrary net charge to form a pseudo-coacervate. This is limited to bioactive materials which form coacervate or pseudo-coacevates with gelatin, a criterion which tends to exclude low molecular weight bioactive materials. Importantly, the coacervated material needs to protect the bioactive from the adverse effects both of gastric pH and gastric enzymes - this requires significant optimisation and testing, and may not be achievable at all.

US Patent No. 5780434 (A. Fjellestad-Paulsen) filed in 1995, describes a composition for oral administration of small and medium sized peptides, particularly vasopressin, oxytocin and their analogues, said composition comprising peptide, a protease inhibitor, and a carrier comprising a buffering agent buffering at a pH of about 5, wherein said mixture is in the form of spheres smaller than 2 mm, said spheres being coated with polymers having dissociable carboxyl groups (enteric coating). Difficulties arise with enteric coating as described previously. The patent provides no information about a desirable gastric pH end value, or pH end value range.

US Patent No. 5525634 describes a matrix-drug combination, wherein the matrix contains a saccharide-containing polymer. The polymer is resistant to chemical and enzymatic degradation in the stomach and is susceptible to enzymatic degradation in the colon by colonic bacteria. This patent provides no teaching on pH modulation in the gastric environment.

International Publication WO 03/080082 of PCT/AU03/00348 (Rawlin and Lichti) describes a method of improving the viability of a labile bioactive substance in a hostile environment, comprising forming a mixture of the bioactive substance and mammalian colostrum. This patent provides no teaching on the protective effect of any material other than colostrum.

Skim milk and/or egg yolk is known to be effective in preserving the viability of spermatozoa in hostile environments such as freezing and thawing. (Squire et al (2004) in Theriogenology Sep 15; 62(6):1056-65).

There is a need for a composition for pH sensitive bioactive materials which provides good bioavailability to a wide range of individuals having diverse gastric environments.

### Summary

Through our studies of bioavailability of pH sensitive materials we have developed an understanding of the requirements for such compositions. We have found that part of the requirements for such a composition is to provide good alkalising activity to acidic gastric liquors, however it is a significant advantage if the composition does not cause neutral gastric environments to become alkaline beyond pH 8.5, and preferably beyond pH 8. We believe the latter constraint arises because gastric alkaline surges (even transient surges) can cause the digestive enzyme pepsin to be permanently inactivated (The Pharmacological Basis of Therapeutics - Goodman and Gillman, 5th edition, p 960). The requirement to meet both of the above constraints (alkalising effect at low pH, non-alkalising effect at neutral pH) does not arise in the formulation of antacids, because antacids are only taken by people with acidic (pH less than 4) gastric liquors.

We have found that the optimal presentation of pH labile bioactives to a gastric environment may involve maintaining the gastric pH between pH 4 and 5, rather than at higher pH values (eg pH 7 or 8) - this is because the rate of gastric acid replenishment increases markedly at higher pH levels.

We have found that by using a carboxylic acid containing moiety and alkali containing moiety and selecting quantities of each it is possible to protect bioactive materials in individuals having a very acid gastric environment at the time of administration whilst also avoiding gastric alkali surges in individuals with less acidic or neutral gastric environment at the time of administration. In this way the bioavailability of the bioactive agent can be improved without the risk of damaging the bioactive agent or inactivating pepsin.

Accordingly we provide in a first aspect of the invention a bioactive agent composition comprising
(a) pH sensitive bioactive agent
(b) an edible carboxylic acid containing moiety and
(c) an edible alkalising moiety,
   wherein the proportion of said moieties and active agent provide pH control such that (i) when 400mg of said composition is added to 20 ml of 0.033 normal hydrochloric acid and at a temperature of 37 +/- 3°C, the pH reaches a value in the range 4 to 8, and (ii) when 400mg of said composition is added to 20 ml of deionised water at pH 7 and at a temperature of 37 +/- 3°C, the pH reaches a value less than 8.5.

In a further aspect the invention provides use of a pH sensitive bioactive agent in preparation of a medicament for oral administration comprising forming a mixture of the pH sensitive bioactive agent with (a) an edible carboxylic acid containing moiety and (b) an edible alkalising moiety, wherein the composition is formulated to react so that (i) when 400mg of said composition is added to 20 ml of 0.033 normal hydrochloric acid and at a temperature of 37 +/- 3°C, the pH reaches a value in the range 4 to 8, and (ii) when 400mg of said composition is added to 20 ml of deionised water at pH 7 and at a temperature of 37 +/- 3°C, the pH reaches a value less than 8.5.

In a further aspect the invention provides a unit dosage composition comprising a pH sensitive bioactive agent and an edible carboxylic acid containing moiety and an edible alkalising moiety wherein: (i) when said unit dosage is added to 20 ml of 0.033 normal hydrochloric acid at a temperature of 37°C +/- 3°C the pH reaches a value in the range 4 to 8; and (ii) when said unit dosage is added to 20 ml of deionised water at pH7 and at a temperature of 37°C +/- 3°C the pH reached a value less than 8.5.

We have surprisingly found that the resulting compositions of the invention provide a composition of the pH sensitive bioactive which is stable to a wide population of individuals having a wide variety of gastric resting volumes and gastric pH values, which in practice cannot be known prior to individual dosing.

The term moiety where used herein refers to a chemical functional group or segment of a molecule or entire molecule. This molecule may be a molecule which may be a molecule of relatively low molecular weight or a macromolecule such as a protein.

The term agent where used herein refers to a chemical entity such as a molecule or substance.

Throughout the description and the claims of this specification the word "comprise" and variations of the word, such as "comprising" and "comprises" is not intended to exclude other additives, components, integers or steps.

Where referred to herein in the specification and claims pH values reported for addition of solid compositions to aqueous materials are determined by adding the solid material in finely divided form to the aqueous composition (at less than 10% w/v), stirring for 30 minutes at 37°C and measuring pH using a glass electrode calibrated for 37°C (This is explained in further detail in the section of the Examples headed "pH protocol").

### Detailed Description

In the preferred aspect of our invention the composition comprises a a pH sensitive bioactive agent and further comprises an edible carboxylic acid containing moiety and an edible alkalising moiety, preferably so that (i) when 400mg of said formulation is added to 20 ml of 0.033 normal hydrochloric acid, the pH reaches a value in the range 4 to 8, and (ii) when 400mg of said formulation is added to 20 ml of deionised water at pH 7, the pH reaches a value less than 8.0.

The bioactive composition comprises a pH sensitive bioactive agent. The bioactive agent is considered pH sensitive if it loses 50% or more activity (preferably at least 75% activity) when 100 mg of the active is continually mixed with 20 ml of 0.033 normal hydrochloric acid for 30 minutes.

The pH sensitive bioactive agent may be selected from the group including vitamins, nutritional supplements, growth promoters, antineoplastic agents, oral vaccines, inhalants, living microorganisms (for example protobiotics such as Lactobacillus spp), peptides, polypeptides, nucleotides, polynucleotides, nucleosides, proteins, glycoproteins, sugars and complex carbohydrates, anti-infectants, antimicrobials, disinfectants, antiseptics, antidepressants, psychoactive agents, genetically modified organisms and infectious agents used as vectors for other bioactive substances eg bacterial vectors (including E. coli, Salmonella, Vibrio, Lactobacilli, Bacillus, Mycobacteria, Shigella), viral vectors (including Adenovirus, Poxvirus, Bacculovirus, Herpesvirus, Enterovirus, Paramyxovirus and Orthomyxovirus), plant vectors (including tobacco, potato and banana), yeast vectors, immunoglobulins, affinity purified immunoglobulins including antibodies directed against diseases and disease causing agents (for example Helicobacter pylori, E. coli, Bacillus spp, pathogenic Yersinia spp., and allergens) and fragments, derivatives and complexes containing any of the above.

The edible carboxylic acid containing moiety may comprise one or more selected from the group consisting of: acetic acid; ascorbic acid; polyacid moieties such as citric acidand tartaric acid; amino acids; peptide chains or proteins; alginic acid; polyacrylic acid; polymethacrylic acid; and copolymers of one or more monomers selected from the group of acrylic acid methacrylic acids; and carboxylic acid containing cellulose derivatives. The edible carboxylic acid may have nutritional value, for example a protein or protein fragment derived from the processing of dairy fluids or vegetables or meats.

The edible alkalising moiety can be any moiety which at 400mg dose is capable of elevating the pH of 20 ml of 0.033 normal hydrochloric acid to a final pH of 4 or greater. Preferably the edible alkalising moiety is capable of elevating the pH of 20ml 0.033 normal hydrochloric acid to a final pH of 5 or more, or even 6 or more. This moiety may comprise an alkali agent such as selected from an alkaline phosphate salt, an alkaline carbonate, an alkaline bicarbonate salt, a hydroxy salt and mixtures of two or more thereof. The alkaline moiety may comprise at least one of the salts selected from the group consisting of the calcium and magnesium salts of one or more of carbonate, bicarbonate, silicate salts and magnesium carbonate co-precipitate. The alkalising agent may be in the form of other basic salts comprising nitrate, carbonate or gallate moieties. The alkalising moiety may comprise a weak acid containing moiety which has been reacted with an alkali such as an amine containing alkali or an alkali such as at least one of potassium hydroxide, lithium hydroxide, sodium hydroxide, aluminium hydroxide, calcium hydroxide, magnesium hydroxide, and aluminium oxide.

It is possible that the edible carboxylic acid containing moiety (which may be in protonated or de-protonated form) and the edible alkalising moiety are one and the same that is part of the same substance. For example, if acid groups in a protein or polycarboxylic acid are partially pre-reacted with sodium hydroxide or an amine-containing base then the resulting product will contain both moieties.

The carboxylic acid containing moiety may be present in the bioactive agent. For example, the bioactive agent may comprise macromolecules such as proteins which have acid and/or alkali moieties and may in some cases be modified to provide the appropriate balance of the required moieties.

In one preferred embodiment, the bioactive agent comprises: a hyperimmune fraction derived from bovine colostrum or avian egg yolk. The hyperimmune fraction may for example be a hyperimmune material directed against enteric bacteria, enteric viruses, anthrax, plague, oral bacteria, respiratory bacteria, food borne bacteria.

In another preferred embodiment the bioactive agent comprises hyperimmune colostrum harvested from dairy cows vaccinated with a vaccine comprising one or more cell wall antigens reactive in a manner characteristic of O group serotypes from enteric disease causing Gram negative bacteria. These colostrum moieties and the associated vaccines, together with more preferred embodiments, are described in our copending International Application PCT/AU 2004/00277 (published as WO 2004/078209), the contents of which are incorporated by reference.

In another preferred embodiment the bioactive agent comprises lactoferrin or lactoferracin.

In another preferred embodiment the formulation of this invention comprises normal colostrum and hyperimmune colostrum harvested from cows vaccinated with a vaccine comprising one or more cell wall antigens reactive in a manner characteristic of O group serotypes from enteric disease causing Gram negative bacteria, wherein the ratio of normal colostrum to hyperimmune colostrum is greater than 1 to 1, preferably greater than 2 to 1, more preferably greater than 3 to 1.

In a particularly preferred embodiment the edible alkaline moiety is a relatively insoluble alkali such as calcium or magnesium carbonate.

The unit dosage composition may be in the form of a tablet, capsule, caplet, syrup or other suitable form. Preferably the unit dosage is in the form of a tablet, capsule or caplet. Typically the unit dosage will contain in the range from 50 to 700 mg of composition and more preferably from 100 to 500 mg of composition.

The carboxylic acid containing moiety preferably is a dairy derived protein such as colostrum or milk or a fraction or concentrate or enzymic or non-enzymic hydrolysate thereof and the edible alkalising moiety is calcium or magnesium carbonate. The dairy derived protein and the calcium or magnesium carbonate may be co-added as a mixed powder fill in a capsule or tablet, or these materials may be co-suspended in an aqueous liquor which is subsequently dried and processed to form a powder.

The dairy concentrate preferably contains greater than 80% protein.

Preferably the weight ratio of dairy derived protein to calcium or magnesium carbonate is greater than 2 to 1, more preferably greater than 3 to 1, still more preferably greater than 5 to 1.

The invention will now be described with reference to the following examples. It is to be understood that the examples are provided by way of illustration of the invention and that they are in no way limiting to the scope of the invention.

### Examples

### pH Protocol

Where referred to herein in the specification and claims pH values reported for addition of solid compositions to aqueous materials are determined by:
(a) adding simulated gastric liquor ( unless otherwise specified 20 ml of .033N hydrochloric acid of nominal pH 1.5) or distilled water (unless otherwise specified 20 ml) to a 50 ml Falcon tube, and incubating in a 37 degrees centigrade water bath for 10 minutes,
(b) adding finely divided or ground up solid material (unless othrewise specified 400 mg) to the Falcon tube and vortexing for 5 seconds,
(c) taping the Falcon tube to one extremity of a Ratek Rocking Platform mixer (model ERPM4, sold by Ratek Instruments, Boronia, Victoria, Australia), which rocks through 15 degrees of arc and is 35 cm in length from one extremity to another, in such a manner that the long axis of the tubes also rock through 15 degrees of arc,
(d) rocking the samples at 50 rpm for 30 minutes, with the rocking platform and attached samples located inside a 37 degrees centigrade incubator.
(e) after rocking and incubation measuring the pH of the contents of the Falcon tube using a pH meter. The pH meter is calibrated between each sample to be measured.

**Example 1: Lactobacillus plantarum - Sample composition** Samples were made having the composition described in the following table - the components were finely divided and mixed together. The bioactive was L. *plantarum* (freeze-dried powder, see example 5).

**Table 1**

| Sample id | Parts bioactive | Parts carboxylic moiety | Parts alkalising moiety |
|---|---|---|---|
| Lp1 | 100 | 400 (colostrum) | - |
| Lp2 | 100 | 378 (colostrum) | 22 (calcium carbonate) |
| Lp3 | 100 | - | 400 (calcium carbonate) |
| Lp4 | 100 | - | 400 (sodium carbonate) |
| Lp6 | 100 | 320 (colostrum) | 80 (sodium carbonate) |
| Lp7 | 100 | 320 (colostrum) | 80 (calcium carbonate) |
| Lp8 | 100 | 220 (citric acid) | 180 (calcium carbonate) |
| Lp9 | 100 | 270 (citric acid) | 130 (calcium carbonate) |

**Example 2: Performance of samples described in example 1** 400 milligrams of sample was added to 20 ml 0.033N HCl and to 20 ml distilled water. Measurements were taken of the final pH (see section on pH protocol) and of the viability of the L.plantarum after the treatment, expressed as cfu per 20ml (see example 6). The results are tabulated below (EXP5 means 100,000, etc):

**Table 2**

| Sample id | PH (after acid treatment) | Viability (after acid treatment) | PH (after water treatment) | Viability (after water treatment) |
|---|---|---|---|---|
| Lp1 | 2.2 | 0 | 6.3 | 4.5EXP6 |
| Lp2 | 3.7 | 1.6EXP5 | 6.9 | 5.2EXP6 |
| Lp3 | 6.6 | 4.4EXP6 | 8.8 | 3.1 EXP6 |
| Lp4 | 9.3 | 2.2EXP6 | 9.6 | 2.1 EXP6 |
| Lp6 | 6.9 | 6.0EXP4 | 9.2 | 2.7EXP6 |
| Lp7 | 5.9 | 3.6EXP6 | 6.9 | 5.9EXP6 |
| Lp8 | 4.75 | 6.4EXP6 | 5.98 | 5.7EXP6 |
| Lp9 | 2.15 | 0 | 2.27 | 0 |

Discussion:
Sample Lp1 is not a composition according to this invention - the pH after acid treatment (representative of a resting gastric pH) was 2.2 (well below the limit of 4 for the invention), and the L.plantarum was not viable.

Sample Lp2 is not a composition according to this invention - the pH after acid treatment is 3.7 (marginally below the limit of 4 for the invention). The L.plantarum viability is down by a factor of 40 on the water treatment.

Sample Lp3 is not a composition according to the invention - the pH after acid treatment is above 4 however the pH after water treatment is above 8.5. Whilst the L.plantarum is viable, the final pH is above 8.5, and it would be expected that the ingestion of this formulation, particularly on a prolonged repetitive basis would promote gastric acid secretion or undesirable gastric alkalinity in people with relatively neutral gastric environments.

Sample Lp4 is not a composition according to the invention - the final pH after acid and water treatment is over 8.5 and it would be expected that that the ingestion of this formulation, particularly on a prolonged repetitive basis would promote gastric acid secretion or undesirable gastric alkalinity in people with relatively neutral gastric environments.

Sample Lp6 is not a composition according to this invention - the final pH after water treatment is over 8.5 and it would be expected that that the ingestion of this formulation , particularly on a prolonged repetitive basis would promote undesirable gastric alkalinity in people with relatively neutral gastric environments.

Sample Lp7 is a composition according to the invention - the final pH after acid treatment is over 4, and the final pH after water treatment is less than 8.5, and the L.plantarum is viable.

Sample Lp8 is a composition according to this invention - the final pH after acid treatment is over 4, and the final pH after water treatment is less than 8.5, and the L.plantarum is viable.

Sample Lp9 is not a composition according to the invention - the final pH after acid treatment is less than 4 and the L.plantarum is not viable.

**Example 3: Erythromycin, urease sample compositions** The bioactive materials in this example were erythromycin and Urease (see example 7).

**Table 3**

| Sample id | Parts bioactive material | Parts component 1 | Parts component 2 |
|---|---|---|---|
| Er1 | 1.0 (erythro) | 320 (colostrum, carboxyl moiety) | 80 (calcium carbonate, alkalising moiety) |
| Er2 | 1.0 (erythro) | 400 (sucrose, blank moiety) | - |
| Ur1 | 168 (urease) | 320 (colostrum,carboxyl moiety) | 80 (calcium carbonate, alkalising moiety) |
| Ur2 | 168 (urease) | 400 (sucrose,blank moiety) | - |

**Example 4: Performance of samples described in Example 3**

**Table 4**

| Sample id | PH (after acid treatment) | Relative activity (after acid treatment) | PH (after water treatment | Relative activity (after water treatment) |
|---|---|---|---|---|
| Er1 | 5.8 | 65% | 7.0 | 95% |
| Er2 | 1.7 | <5% | 6.8 | 100% |
| Ur1 | 6.0 | 75% | 76.9 | 105% |
| Ur2 | 1.8 | <5% | 6.9 | 100% |

Discussion:
Sample Er1 is a composition according to the invention - the pH after acid treatment is over 4 and the pH after water treatment is less than 8.5. The relative activity of erythromycin is substantial after both treatments.

Sample Er2 is not a composition according to the invention - there are no carboxyl components, no alkalising components, and low relative activity of bioactive after acid treatment. The activity of bioactive in this sample after water treatment has been used to set the 100% level.

Sample Ur1 is a composition according to the invention - the pH after acid treatment is over 4 and the pH after water treatment is less than 8.5. The relative activity of urease is substantial after both treatments.

Sample Ur2 is a not a composition according to the invention - there are no carboxyl components, no alkalising components, and low relative abundance of bioactive after acid treatment. The activity of bioactive in this sample after water treatment has been used to set the 100% level.

Example 5: Preparation of L.plantarum freeze-dried powder Freeze-drying media (FDM) was prepared as follows (method derived from Conrad PB et al. (2000) in Stabilisation and preservation of Lactobacillus acidophilus in saccharide matrices. Cryobiology 41, 17-24): Trehalose dihydrate (Sigma) and sodium tetraborate decahydrate (Sigma) were dissolved in sterile 0.6mM potassium phosphate pH 7.2 at 40%w/v and 5.7%w/v respectively. The pH was adjusted to 6.5 with solid citric acid (Sigma) and then to 8.5 with ammonium hydroxide (Sigma 29.5% solution).

*L.plantarum* used in this study was taken from the culture collection of the Microbial Research Unit Royal Childrens Hospital, Parkville, Victoria, Australia, and typed by 16S sequencing (sequencing of DNA from ribosomal subunits). The strain was grown as a lawn culture (20 plates) on MRS agar at 37deg C in an incubator with a 5% CO₂: air mix for 48 hrs. The culture was scraped off the 20 plates and combined in 20 mls saline 0.85%. The saline liquor was centrifuged for 15 min at room temperature, and the pellet was resuspended in 5mls saline. To this 5ml aliquot was added 5ml FDM, with vortexing, and the combined liquor was freeze-dried in a Dynavac "Mini Ultra Cold" freeze-dryer. Max vacuum from pump = 0.1mbar; vacuum (steady state) of freeze-drying chamber = 0.38mbar; working temp = -100deg C; volume (condenser capacity) = 1.7 L. The freeze-drying process was run over-night (18hrs) until the vacuum approached 0.38mbar. The freeze-dried material was ground into a fine powder and added to other sample components.

**Example 6: Determination of L.plantarum viability.** After acid or water treatment in the 50 ml Falcon tube (20ml liquor volume), 250 microlitres of liquor was taken (duplicates) and the liquor quenched by the addition of 62 microlitres of chilled 200mM Tris-HCl pH 8.0. *L. plantarum* survival was assayed using a viable plate count technique. Ten-fold dilutions of the incubation mixture were prepared in MRS broth immediately after quenching. 100 microlitres of each dilution were then spread onto duplicate plates. Plates were incubated for 48 hrs and the number of colonies per plate counted. The number of colony forming units in the incubation mixture was then calculated by multiplying the number of colony forming units of diluted suspension by the dilution factor.

**Example 7: Preparation /Acquisition of Other Components**
Urease was Sigma Jack Bean Urease Type III (Cat No U-1500). This freeze-dried powder had a quoted activity of 16 units per mg (one unit liberates 1.0 micro-moles of ammonia from urea per min at pH 7.0 and 25 deg C).
**Erythromycin** powder was from Boehringer Mannheim.

**Colostrum** The following diagram shows the principles used to take colostrum and convert it to a processed form.

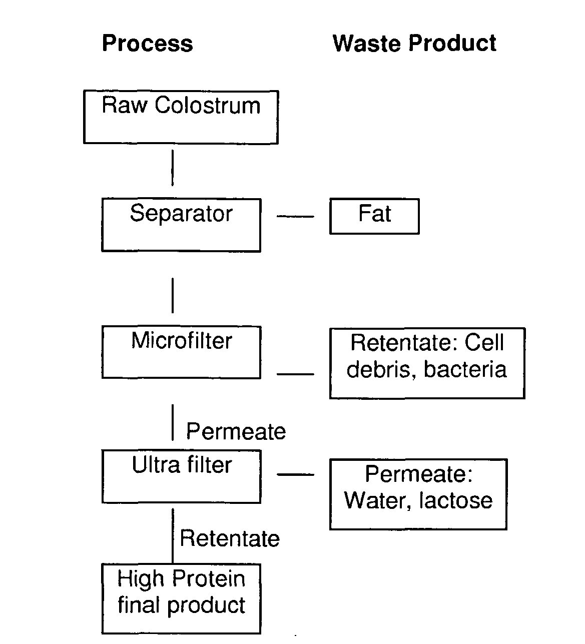

The raw colostrum is collected from dairy cows most preferably at the first milking after calving. The colostrum is stored at 4°C on farm and then transported either for longer term storage at -200C or sent directly to wet manufacturing.

The raw colostrum is warmed to approximately 37°C and then skimmed with a rotary milk separator to remove fat. The resultant liquid may be pasteurised or microfiltered with a 7-10 micron ceramic filter system (to remove bacteria and debris. The liquid is then Ultrafiltered (for example in a Abcor 10m² Ultrafiltration plant) to remove a majority of the water, lactose and electrolytes leaving a high protein concentrate. The resultant high protein concentrate is further processed preferably by lyophilization (freeze-drying) or spray-drying.

The above method yield a processed bovine colostrum powder with the specifications as below. This product as defined below is listed as a substance suitable for inclusion in therapeutic goods by the Therapeutic Goods Authority of Australia.

**Definition:** Bovine colostrum powder is derived from the first milking of Australian, or New Zealand* cows (Bos taurus) following concentration and lyophilisation.
**Appearance:** Free-flowing, pale yellow powder.
**Properties:** Soluble in water. Mild odour of milk when contacted with moisture.
**Moisture** Range 2 to 5 % m/m AS 2300.1.1 (1988)
**Fat** Range 1 to 4 % m/m AS 2300.1.3 (1988)
**Ash** (@550°C) Not more than 8 % m/m AS 2300.1.5 (1988)
**Total Nitrogen** (TN) For information** AS 2300.1.2 (1991)
**Non-protein nitrogen(NPN)** For information** AS 2300.1.2.2 (1988)
**True protein** Not less than 60 % m/m (TN-NPN)% x 6.38
**Protein** Not less than 60 % m/m AS 2300.1.2 (1991)
**Lactose (monohydrate)** Not more than 15 % m/m
UV assay following enzymatic hydrolysis and oxidation
(Boehringer Mannheim)
**Total immunoglobulins** Not less than 20 % m/m
Radial immunodiffusion assay
**Microbial limits** Complies with TGA guidelines
**Residues:** Heavy metals Agricultural and Veterinary chemicals

Subject to ANZFA Food Standards Code for dairy products. Where there is no applicable Food Standard, the BP test for heavy metals applies (2 ppm calculated as lead) and also the BP requirements for pesticide residues.

* These countries being BSE-free. Colostrum powder from other countries will require pre-clearance from the TGA ** Used to calculate the value for true protein

'AS' refers to document of the Australian Standards Organisation series of 'Australian Standards' - in this case referring to standardised methods of quality and component testing for dairy products.

**Example 8: Measurement of activity for Erythromycin**
Erythromycin activity was assayed using a bacillus subtilis disc diffusion susceptibility test (Barry AL and Thornsberry C ,1991, Susceptibility tests: diffusion test procedures. In Balos A, Hauser WJ, Herman KL, Isenberg HD, and Shadomy HJ, Manual of Clinical Microbiology 5th Edition, American Society for Microbiology, Washington pp1117-1125). An inoculum of B.subtilis (ATCC 6633) was prepared by picking at least 2 colonies from an overnight culture grown on horse blood agar (HBA) and inoculating 2ml of saline to reach a turbidity equivalent to a 0.5 McFarlane standard. HBA plates for the assay were then inoculated by streaking a sterile swab, dipped into the standardised solution, evenly in three directions over the entire surface of the plate to obtain a uniform inoculum. Plates were then allowed to dry for 3 to 5 minutes before the discs were applied.

An aliquot of erythromycin liquor (after acid or water treatment) was serially diluted 1:2 with MilliQ water, resulting in six dilutions for each. 20 microlitres of each dilution was then loaded into duplicate blank susceptibility discs (Oxoid, Hampshire, England). These discs were allowed to dry for at least 30 minutes before being placed onto duplicate plates. Each plate contained six evenly placed discs corresponding to the six dilutions of a single treatment. Sample Er2 (reacted with water according to the pH protocol) was used as a control to obtain a standard curve. Plates were incubated for 16-18 hours at 37 deg C.

After 16-18 hours incubation the susceptibility of B. subtilis to erythromycin was determined by measuring the diameter of the zones of inhibition which appear around the discs. These zones result from the diffusion of the antibiotic from the disc into the surrounding agar. A standard curve was generated using the diameters of zones resulting from the serially diluted erythromycin control. Diameters from the test samples were then used to obtain thepercentage of erythromycin activity remaining compared with the untreated control.

**Example 9: Measurement of activity for Urease**
250 micro-litre aliquots were taken after sample treatment and the aliquots were quenched by the addition of 0.25 volumes of chilled 160mM Na2CO3. Aliquots were then centrifuged for 3 minutes at 20,000g and stored on ice. Supernatants were assayed for urease activity.

Urease activity was assayed using a coupled enzyme assay for increased sensitivity (modified from Kaltwasser and Schlegel, 1966, NADH-dependent coupled enzyme assay for urease and other ammonia-producing systems. Analytical Biochem, 16:132-138). In this reaction, the urease enzyme catalyses the hydrolysis of urea:
Urea + H₂O + 2H⁺ ⇒ 2NH⁴⁺ + CO₂
which is measured by coupling ammonia production to a glutamic dehydrogenase reaction:
2NH4+ + 2 α-ketoglutarate + 2 NADH ⇒ 2 glutamate + 2NAD+ + 2H2O
the reaction is followed by the oxidation of NADH to NAD.

The final assay volume of 1 ml contained final concentrations of 1.6mM α-ketoglutarate (Boehringer Mannheim Cat No.127 205), 1.5mM NADH (Sigma β-NADH Cat No. N-8129), 15 units/ml of L-glutamic dehydrogenase (Sigma Cat No. G-4387), 10mM Urea (Boehringer Mannheim Cat No. 100 164) and 1 mM sodium sulphide (Sigma Cat No S-4766) in 50mM Tris-HCl buffer (pH 8.0). These reagents were mixed in 1 cm path length polystyrene cuvettes (Sarstedt Cat No 67.742) and then allowed to equilibrate for several minutes to room temperature in a Beckman DU70 recording spectrophotometer. The spectrophotometer was zeroed using the above mixture, before the addition of the sample.

A ten-microlitre sample of the supernatant of each incubation mixture was added to the assay mixture to start the assay. The reaction rate was recorded every 10 seconds at 340nm for up to 4 min at RT. Reaction rate was calculated from the linear portion of curve (generally after first 1-2 mins) and urease activity was then noted as µmole of urea hydrolysed per min per mg of protein. Sample Ur2 was used as the control.

**Example 10 - Bioactives Erythromycin, Lactoferrin: sample composition**

**Table 5**

| Sample ID | Parts Bioactive | Parts Carboxylic moiety | Parts Alkalising moiety |
|---|---|---|---|
| S1 | 1 (Ery) | 320 (Colostrum) | 62 (NaOH) |
| S2 | 1 (Ery) | 320 (Colostrum) | 31 (NaOH) |
| S3 | 1 (Ery) | 320 (Colostrum) | 15.5 (NaOH) |
| S4 | 1 (Ery) | 320 (Colostrum) | 7.8 (NaOH) |
| S5 | 1 (Ery) | 320 (Colostrum) | 111 (Na₂HPO₄) dihydrate |
| S6 | 1 (Ery) | 320 (Colostrum) | 55.5 (Na₂HPO₄) dihydrate |
| S7 | 1 (Ery) | 320 (Colostrum) | 27.5 (Na₂HPO₄) dihydrate |
| S8 | 40 (lactoferrin) | 320 (Colostrum) | 40 (CaCO₃) |
| S9 | 20 (lactoferrin) | 320 (Colostrum) | 60 (CaCO₃) |
| S10 | 10 (lactoferrin) | 320 (Colostrum) | 70 (CaCO₃) |
| S11 | 10 (lactoferrin) | 320 (Colostrum) | 80 (CaCO₃) |
| S12 | 10 (lactoferrin) | 320 (Colostrum) | 90 (CaCO₃) |
| S13 | 10 (lactoferrin) | 320 (Colostrum) | 100 (CaCO₃) |
| S14 | 1 (Ery) | 8 Citric acid dihydrate | 390 (Na₂HPO₄) dihydrate |
| S15 | 1 (Ery) | 250 Citric acid dihydrate | 150 (NaOH dry) |

**Example 11 - Performance of samples described in Example 10**

**Table 6**

| Sample ID | pH (after acid treatment) | pH (after water treatment) |
|---|---|---|
| S1 | 11.5 | 11.7 |
| S2 | 8.4 | 11.7 |
| S3 | 3.3 | 11.4 |
| S4 | 2.5 | 10.5 |
| S5 | 5.5 | 7.6 |
| S6 | 3.2 | 7.8 |
| S7 | 2.5 | 7.0 |
| S8 | 3.8 | 6.9 |
| S9 | 4.2 | 7.0 |
| S10 | 4.6 | 7.0 |
| S11 | 4.9 | 7.0 |
| S12 | 5.2 | 7.1 |
| S13 | 5.4 | 7.1 |
| S14 | 6.6 | 8.2 |
| S15 | 5.6 | 6.7 |

In the above example S5, S9, S10, S11, S12, S13, S14 and S15 are compositions according to the invention.

## Claims

1. A bioactive composition comprising:
(a) pH sensitive bioactive agent
(b) an edible carboxylic acid containing moiety and
(c) an edible alkalising moiety,
wherein the proportion of said moieties and active agent provide pH control such that (i) when 400mg of said composition is added to 20 ml of 0.033 normal hydrochloric acid and at a temperature of 37 +/- 3°C, the pH reaches a value in the range 4 to 8, and (ii) when 400mg of said composition is added to 20 ml of deionised water at pH 7 and at a temperature of 37 +/- 3°C, the pH reaches a value less than 8.5.

2. A bioactive composition according to claim 1 wherein when the 400mg of said composition is added to 20 ml of deionised water at pH 7, the pH reaches a value is less than 8.0.

3. A bioactive composition according to any one of the previous claims comprising the bioactive agent and at least one pH regulating agent component comprising at least one of said alkalising moiety and said carboxylic acid containing moiety wherein the other of said alkalising moiety and said carboxylic acid containing moiety is present in at least one of said bioactive agent and said pH regulating agent component.

4. A bioactive composition according to any one of the previous claims comprising a bioactive agent, and a pH regulating agent comprising both said edible carboxylic acid containing moiety and said edible alkalising moiety.

5. A bioactive composition according to claim 1 wherein the composition comprises the bioactive agent, an alkalising agent comprising the alkalising moiety and an agent comprising the carboxylic acid moiety.

6. A bioactive composition according to any one of the previous claims wherein the bioactive agent is selected from the group consisting of vitamins, nutritional supplements, growth promoters, antineoplastic agents, oral vaccines, inhalants, living microorganisms, peptides, polypeptides, nucleotides, polynucleotides, nucleosides, proteins, glycoproteins, sugars and complex carbohydrates, anti-infectants, antimicrobials, disinfectants, antiseptics, antidepressants, psychoactive agents, genetically modified organisms and infectious agents used as vectors for other bioactive substances eg bacterial vectors (including E. coli, Salmonella, Vibrio, Lactobacilli, Bacillus, Mycobacteria, Shigella), viral vectors (including Adenovirus, Poxvirus, Bacculovirus, Herpesvirus, Enterovirus, Paramyxovirus and Orthomyxovirus), plant vectors (including tobacco, potato and banana), yeast vectors, immunoglobulins, affinity purified immunoglobulins including antibodies directed against diseases and disease causing agents (for example Helicobacter pylori, E. coli, Bacillus spp, pathogenic Yersinia spp., and allergens) and fragments, derivatives and complexes containing any of the above.

7. A bioactive composition according to any one claims 1 to 6 wherein the bioactive agent is selected from the group consisting of growth promoters, oral vaccines, probiotic microrganisms, antimicrobials, bacterial vectors, immunoglobulins, antibodies and antibody fragments.

8. A bioactive composition according to any one of the previous claims comprising a pH regulating agent comprising said carboxylic acid containing moiety comprising at least one substance selected from the group consisting of acetic acid, polyacid moieties, amino acids, or peptide chains, proteins, or alginic acid, polyacrylic acid, polymethacrylic acid, copolymers of one or both of acrylic and methacrylic acids and carboxyl containing cellulose derivatives.

9. A bioactive composition according to any one of the previous claims comprising a pH regulating agent having a carboxylic acid containing moiety comprising at least one of colostrum, citric acid and tartaric acid.

10. A bioactive composition according to claim 8 wherein the acidic agent comprising the carboxylic acid moiety is bovine colostrum.

11. A composition according to any of the previous claims wherein the 400mg of said composition contains sufficient edible alkalising moiety to elevate the pH of 20ml 0.033 normal hydrochloric acid to a final pH of 4 or more, more preferably to a final pH of 5 or more.

12. A composition according to any of the previous claims wherein the composition comprises a pH regulating agent comprising an alkalising moiety selected from the group consisting of alkaline phosphate salts, alkaline carbonate salts, alkaline bicarbonate salts, hydroxy salts and mixtures of two or more thereof.

13. A bioactive composition according to any one of the previous claims comprising an edible alkalising agent comprising the alkalising moiety and selected from at least one of calcium carbonate, magnesium carbonate, magnesium bicarbonate, silicate salts, and basic salts comprising nitrate, carbonate or gallate moieties.

14. A bioactive composition according to any one of the previous claims wherein the alkalising moiety comprises a weak acid containing moiety which has been reacted with an alkali selected from the group consisting of amine containing alkali, potassium hydroxide, lithium hydroxide, aluminium hydroxide, calcium oxide or hydroxide, magnesium oxide or hydroxide and aluminium oxide or hydroxide.

15. A bioactive composition according to any one of the previous claims wherein the bioactive agent comprises at least one substance selected from the group consisting of antibodies derived from hyperimmune bovine colostrum or hyperimmune avian egg yolk.

16. A bioactive composition according to claim 15 wherein the antibodies are directed against at least one antigen selected from the group selected from enteric bacteria, enteric viruses, anthrax, plague, oral bacteria, respiratory bacteria and foodborne bacteria.

17. A composition according to any one of the previous claims wherein the bioactive agent comprises the hyperimmune fraction of colostrum harvested from dairy cows vaccinated with a vaccine comprising one or more cell wall antigens reactive in a manner characteristic of O group serotypes from enteric disease causing gram negative bacteria.

18. A bioactive composition according to any one of the previous claims wherein the bioactive agent comprises lactoferrin or lactoferracin.

19. A bioactive composition according to anyone of the previous claims comprising as the bioactive agent the hyperimmune fraction of colostrum harvested from cows vaccinated with a vaccine comprising one or more cell wall antigens reactive in a manner characteristic of O group serotypes from enteric disease causing Gram negative bacteria, and a pH regulating agent comprising colostrum wherein the ratio of normal colostrum to hyperimmune colostrum is greater than 1 to 1.

20. A bioactive composition according to claim 19 wherein the ratio is greater than 3 to 1.

21. A bioactive composition according to any one of the previous claims wherein the composition comprises an alkalising agent comprising the edible alkalising moiety selected from calcium and magnesium carbonate.

22. A composition according to claim 1 wherein the composition comprises an agent comprising a carboxylic acid moiety selected from the group consisting of colostrum, milk or a fraction, concentrate or hydrolysate thereof and the edible alkalising moiety is at least one of calcium carbonate and magnesium carbonate.

23. A composition according to claim 22 wherein the weight ratio of dairy derived protein to calcium or magnesium carbonate is greater than 2 to 1.

24. A bioactive composition according to claim 23 wherein the ratio is greater than 4 to 1.

25. A unit dosage composition comprising a pH sensitive bioactive agent an edible carboxylic acid containing moiety and an edible alkalising moiety wherein: (i) when said unit dosage is added to 20 ml of 0.033 normal hydrochloric acid at a temperature of 37°C +/- 3°C the pH reaches a value in the range 4 to 8; and (ii) when said unit dosage is added to 20 ml of deionised water at pH 7 and at a temperature of 37°C +/- 3°C the pH reached a value less than 8.5.

26. A unit dosage composition according to claim 25 wherein the composition is as defined according to any one of claims 1 to 24.

27. Use of a pH sensitive bioactive agent in preparation of a medicament for oral administration comprising forming a mixture of the pH sensitive bioactive agent with (a) an edible carboxylic acid containing moiety and (b) an edible alkalising moiety, wherein the composition is formulated to react so that (i) when 400mg of said composition is added to 20 ml of 0.033 normal hydrochloric acid and at a temperature of 37°C +/- 3°C, the pH reaches a value in the range 4 to 8, and (ii) when 400mg of said composition is added to 20 ml of deionised water at pH 7 and at a temperature of 37°C +/- 3°C, the pH reaches a value less than 8.5.
